(19) Europäisches Patentamt
European Patent Office
Office européen des brevets

(11) Numéro de publication: **0 237 411 A1**

(12) # DEMANDE DE BREVET EUROPEEN

(21) Numéro de dépôt: **87400452.6**

(22) Date de dépôt: **02.03.87**

(51) Int. Cl.[4]: **C 07 D 295/08**
A 61 K 31/445

(30) Priorité: **04.03.86 FR 8603001**

(43) Date de publication de la demande:
**16.09.87 Bulletin 87/38**

(84) Etats contractants désignés:
**AT BE CH DE ES FR GB GR IT LI LU NL SE**

(71) Demandeur: **INSTITUT DE RECHERCHES CHIMIQUES ET BIOLOGIQUES APPLIQUEES (I.R.C.E.B.A.) Société à responsabilité limitée dite:**
**62, Grande-Rue**
**F-78490 Vicq (FR)**

(72) Inventeur: **Danree, Bernard**
**59 bis Boulevard Devaux**
**F-78300 Poissy (FR)**

**Houziaux, Patrick**
**Chemin des Jonchères Bazemont**
**F-78580 Maule (FR)**

**Lacolle, Jean-Yves**
**Résidence du Parc**
**F-78860 Saint Nom La Breteche (FR)**

(74) Mandataire: **Combe, André et al**
**CABINET BEAU DE LOMENIE 55, rue d'Amsterdam**
**F-75008 Paris (FR)**

(54) **(Pipéridino-3)propoxy - 5p.cymène, les dérivés acétyl, acétoxy et hydroxy dudit produit, les sels de ces produits un procédé de préparation desdits produits et les médicaments contenant au moins un de ces produits comme principe actif.**

(57) La présente invention concerne des produits chimiques de formule :

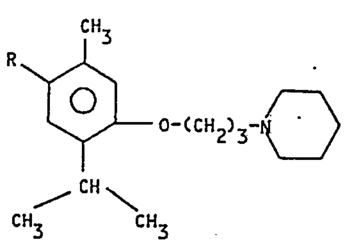

(I)

dans laquelle R est un radical choisi parmi H, acétyl ($COCH_3$), acétoxy ($OCOCH_3$) et hydroxy (OH), ainsi que les sels desdits produits, avec des acides, en vue de la préparation d'un composé pharmaceutiquement acceptable ; - elle concerne également un procédé de préparation de ces produits et les médicaments qui en contiennent.

EP 0 237 411 A1

Bundesdruckerei Berlin

## Description

[(Pipéridino-3)propoxy]-5 p.cymène, les dérivés acétyl, acétoxy et hydroxy dudit produit, les sels de ces produits, un procédé de préparation desdits produits et les médicaments contenant au moins un de ces produits comme principe actif.

La présente invention concerne :

- en tant que produits nouveaux le [(pipéridino-3)propoxy]-5 p.cymène, les dérivés acétyl, acétoxy et hydroxy en 2 de ce produit, les sels de ces divers produits ;
- un procédé de préparation de ces divers produits,
- et les médicaments contenant, en tant que principe actif, au moins un desdits produits.

Les produits de l'invention ont pour formule :

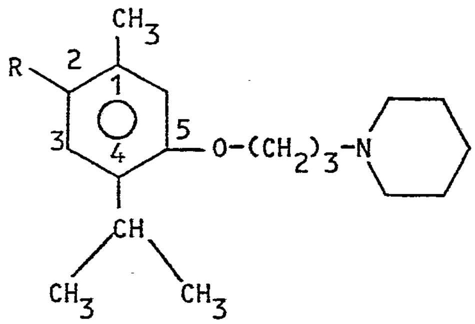

(I)

R étant un radical choisi parmi H, acétyl ($COCH_3$), acétoxy ($OCOCH_3$) et hydroxy (OH).

Les produits de formule (I) peuvent également se présenter sous forme de sels avec des acides pharmaceutiquement acceptables et notamment avec l'acide chlorhydrique.

Le produit de formule (I) dans lequel R est H peut être préparé par réaction du thymol avec la N-(chloro-3-propyl)pipéridine utilisée par exemple sous la forme de son chlorhydrate ; la réaction est réalisée par transfert de phase en système liquide-liquide en présence d'un catalyseur tel que le chlorure de triéthylbenzylammonium.

Le produit de formule (I) dans lequel R est le radical acétyle est réalisé par réaction du produit de formule (I) dans lequel R est H avec de l'anhydride acétique en présence d'acide perchlorique ; cette réaction est avantageusement effectuée dans un solvant tel que le toluène.

Le produit de formule (I) dans lequel R est le radical acétoxy est réalisé par action, sur le dérivé de formule (I) où R est acétyle, d'un oxydant tel que l'acide m-chloroperbenzoïque ; cette réaction est réalisée dans un solvant (par exemple le chlorure de méthylène) en présence d'un acide (par exemple l'acide trichloroacétique).

Le produit de formule (I) dans lequel R est OH est préparé par saponification, à l'aide d'une solution de soude par exemple, du produit de formule (I) dans lequel R est le radical acétoxy.

Comme indiqué ci-dessus, chacun des produits de formule (I) peut se présenter sous forme d'un sel par utilisation d'un acide donnant naissance à un produit pharmaceutiquement acceptable ; la préparation des chlorhydrates, par exemple, peut s'effectuer par simple barbotage d'acide chlorhydrique gazeux dans une solution d'un produit de formule (I).

La présente invention concerne également les médicaments caractérisés en ce qu'ils contiennent, comme produit actif, au moins un produit de formule (I) ; les médicaments obtenus sont utiles notamment dans le domaine de l'urologie.

Les exemples non limitatifs suivants illustrent les procédés de préparation des produits selon l'invention.

### Exemple 1

Synthèse du chlorhydrate du [(pipéridino-3)propoxy]-3 p.cymène (B 1020).

1) [(pipéridino-3)propoxy]-3 p.cymène

Dans un tricol de 4 l, muni d'un réfrigérant, d'une agitation pneumatique et d'un thermomètre, on introduit :

150,2 g (1 mole)de thymol,
16,68 g de chlorure de triéthylbenzylammonium,
751 ml de lessive de soude,
1495 ml de chlorure de méthylène.

On y ajoute 247,67 g (1,25 mole)de chlorhydrate de la N-(chloro-3-propyl)-pipéridine dans 74,2 ml d'eau. Le milieu est ènergiquement agité. Le mélange est chauffé au reflux pendant 5 h sous vive agitation. Après refroidissement à température ambiante, on décante la phase organique. On extrait la phase sodique par 2 × 1,050 l de chlorure de méthylène. Les phases organiques réunies sont lavées successivement par 2 × 1,780

l d'eau acidulée (acide acétique O,25 %), chargée de chlorure de sodium, puis 4 × 1,78O l d'eau saturée en chlorure de sodium (jusqu'à neutralité). On les sèche sur sulfate de sodium. Après filtration, le solvant est chassé sous vide. On obtient 3O2,37 g d'une huile brune. Le produit brut est purifié par distillation fractionnée sous vide (sous azote).

On isole 174,33 g d'une huile jaunâtre présentant :

. une température d'ébullition $Eb_{0,15\ mmHg}$ = 185-19O° C
. des spectres IR et RMN conformes à la formule énoncée,
. une pureté supérieure à 97 %. (C.P.V.)

2) B 1O2O

27,54 g de cette huile (O,1 mole)sont dissous dans 29O ml d'éther éthylique anhydre. La solution refroidie est saturée par un courant gazeux de HCl sec. Les cristaux formés sont filtrés sur verre fritté, lavés par de l'éther éthylique anhydre puis séchés sous vide à 5O° C. On obtient 28,41 g de cristaux blancs (rendement brut : 91,1 %). Après recristallisation dans un mélange AcOEt/IPA (4/1), on isole 24,14 g de cristaux blancs.

Le produit obtenu présente les propriétés suivantes :

. température de fusion $F_{BK}$ = 166-167° C
. titre TBAH = 99,3 %
. spectres IR et RMN conformes à la structure proposée.

Exemple 2

Synthèse du chlorhydrate de l'acétyl-2-[(pipéridino-3)propoxy]-5 p.cymène (B 1O21)

1) Acétyl-2-[(pipéridino-3)propoxy]-5 p.cymène

275,44 g (1 mole)du produit distillé obtenu à la première étape de l'exemple 1 sont dissous dans 1,6 l de toluène et 863 ml d'anhydride acétique dans un tricol de 4 l muni d'un réfrigérant équipé d'une garde à $H_2SO_4$, d'un thermomètre et d'une ampoule à introduction. Le mélange est agité, puis on ajoute goutte à goutte, en maintenant la température inférieure à 45 C, 225,2 ml d'acide perchlorique à 7O %. Le mélange est agité pendant 1 h à température ambiante, puis versé sur 73O ml d'eau saturée en NaCl. Après refroidissement par un bain de glace et basification par de la lessive de soude (pH = 12), on sépare la phase organique, on extrait par 3 × 73O ml de chlorure de méthylène. Les phases organiques sont réunies et lavées avec de l'eau acidulée chargée en chlorure de sodium, puis par 6 × 73O ml d'eau saturée en chlorure de sodium (jusqu'à neutralité). On sèche sur sulfate de sodium, on filtre et on chasse le solvant sous vide. On obtient 245,41 g d'une huile brune.

2) B 1O21

31,75 g (O,1 mole)de cette huile brune sont dissous dans 4OO ml d'éther éthylique anhydre et refroidis par un bain de glace. On y fait barboter un courant gazeux de HCl sec. Les cristaux formés sont filtrés sur verre fritté, lavés par de l'éther éthylique anhydre, puis séchés sur potasse à 5O°C sous vide. On isole 3O,O8 g de cristaux beiges (rendement brut : 85 %). Après recristallisation dans un mélange AcOEt/IPA (6/1), on obtient 17,41 g de cristaux beige clair.

Ces produits ont été analysés et les résultats ci-après ont été obtenus :

. température de fusion $F_{BK}$ = 162-163°C
. titre TBAH : 99 %
. spectres IR et RMN conformes à la formule proposée.

Exemple 3

Synthèse du chlorhydrate de l'acétoxy-2-[(pipéridino-3)propoxy]-5 p.cymène (B 1O59)

1) Acétoxy-2-[(pipéridino-3)propoxy]-5 p.cymène

Le produit obtenu après la première étape de l'exemple 2 est utilisé brut. 317,48 g (1 mole) de ce produit sont introduits avec 1,7OO l de chlorure de méthylène dans un tricol de 4 l muni d'un réfrigérant équipé d'une garde à $H_2SO_4$, d'un thermomètre et d'une agitation pneumatique. 392,13 g (2,4 mole) d'acide trifluoroacétique sont ajoutés par portions, en maintenant la température inférieure à 15°C. On introduit alors 258,84 g (1,2 mole) d'acide m-chloroperbenzoïque à 8O %. Le mélange est maintenu 24 h à 15° C sous agitation. On le verse sur 2,26 l d'ammoniaque à 5 %. On décante la phase organique que l'on sépare. On extrait la phase aqueuse par 2 × 2 l de chlorure de méthylène. Les phases organiques sont rassemblées, lavées avec 2,5 l d'eau acidulée chargée en chlorure de sodium, puis 2 × 2,5 l d'eau saturée en NaCl (jusqu'à neutralité). On sèche sur sulfate de sodium, on filtre, et on chasse le solvant sous vide. On obtient 337,48 g d'une huile brune d'une pureté C.P.V. de 83 % (rendement brut > 1OO %). Ce produit brut est purifié par distillation fractionnée sous vide (sous azote).

On isole 145,39 g d'une huile jaunâtre présentant :

. une température d'ébullition (sous O,2 mmHg) de 2O5-21O° C, et
. des spectres IR et RMN conformes à la formule proposée.

2) B 1059

33,34 g (0,1 mole)de cette huile distillée sont dissous dans 200 ml d'éther éthylique anhydre. La solution refroidie est saturée par un courant gazeux de HCl sec. Les cristaux formés sont filtrés sur verre fritté, lavés par de l'éther éthylique anhydre puis séchés sous vide à 50° C. On isole 33,44 g de cristaux beiges (rendement brut : 90,4 %). Après recristallisation dans l'acétate d'éthyle, on obtient 24,04 g de cristaux beige clair.

Ces cristaux analysés ont donné les résultats suivants :

. température de fusion $F_{BK}$ : 159-160° C
. titre TBAH : 98,4 %
. titre $AgNO_3$ 99,7 %
. spectres IR et RMN conformes à la structure proposée.

Exemple 4

Synthèse du chlorhydrate de l'hydroxy-2-[(pipéridino-3)propoxy]-5 p.cymène (B 1037)

1) Hydroxy-2-[(pipéridino-3)propoxy]-5 p.cymène

333,48 g (1 mol) du produit distillé obtenu après la première étape de l'exemple 3 sont introduits avec 1,1 l d'éthanol dans un tricol de 4 l muni d'un réfrigérant, d'un thermomètre et d'une agitation pneumatique. 1,10 l de soude 1 N (1,10 mol) sont ajoutés à cette solution, et le mélange est agité 24 h à température ambiante. L'éthanol est chassé sous vide ; le résidu est repris par 2,2 l d'eau, extrait par 2 × 2,2 l de chlorure de méthylène. Les phases organiques réunies sont lavées par de l'eau saturée en chlorure de sodium (jusqu'à neutralité). On sèche sur sulfate de sodium, on filtre et on chasse le solvant sous vide. On obtient 259,38 g d'une huile brune (rendement brut : 89 %). Après cristallisation dans le pentane, on isole 244,51 g de cristaux bruns, ayant une température de fusion $F_{BK}$ de 104-106°C.

2) B 1037

29,14 g (0,1 mol) de base purifiée sont dissous dans 1,160 l d'éther éthylique anhydre sous agitation. Après refroidissement de la solution, et barbotage d'un courant gazeux de HCl sec, on isole les cristaux formés par filtration sur fritté. Après lavage par de l'éther éthylique anhydre, séchage sous vide à 50°C, on obtient 30,75 g de cristaux beiges (rendement brut : 93,8 %). Après recristallition dans l'éthanol, on isole 21,87 g de cristaux légèrement beiges.

A l'analyse, ces cristaux présentent les résultats suivants :

. température de fusion $F_{BK}$ 213-215°C
. titre TBAH : 97,5 %
. spectres IR et RMN conformes à la structure proposée.

Les propriétés pharmacologiques des produits obtenus ont été étudiées ; lors de cette étude, le produit de référence choisi a été le chlorhydrate de thymoxamine.

1. Tests de toxicité aiguë chez la souris

Les résultats sont rapportés dans le tableau 1.

TABLEAU 1

| Produits | Mortalité en % | |
|---|---|---|
| | 1.000 mg.kg$^{-1}$ P.O | 100 mg.kg$^{-1}$ P.O |
| B 1020 | 40 | 0 |
| B 1021 | 100 | 20 |
| B 1037 | 100 | 0 |
| B 1059 | 100 | 0 |
| Thymoxamine | 100 | 0 |

2. Détermination de l'action α-adrénolytique "in vitro" sur le canal déférent du rat.

a) Principe de la mesure

La noradrénaline provoque des contractions du canal déférent isolé de rat. La présence dans le bain de l'organe de substances α-bloquantes antagonise ces contractions.

L'utilisation de concentrations croissantes de substances α-bloquantes permet de calculer :

. la $PA_2$ des composés sur le canal déférent de rat.

La $PA_2$ étant le logarithme changé de signe de la concentration molaire des produits B 1020 - B 1021 - B 1037 - B 1059 en présence de laquelle il faut multiplier par deux la concentration de noradrénaline pour obtenir le même effet qu'en l'absence dudit produit.

b) Résultats obtenus

Les résultats sont consignés dans le tableau 2.

TABLEAU 2

| Produit | Action α-bloquante vis-à-vis de la noradrénaline $PA_2$ sur canal déférent isolé |
|---|---|
| B 1020 | 6,52 |
| B 1021 | 5,76 |
| B 1037 | 6,82 |
| B 1059 | 6,90 |
| Thymoxamine | 7,25 |

Ces résultats montrent que les produits testés manifestent une activité α-bloquante intéressante.

3. Détermination de l'activité adrénolytique "in vivo" chez le rat

a) Principe de la mesure

. La noradrénaline injectée à fortes doses par voie intraveineuse provoque la mort de 100 % des animaux dans les 15 min qui suivent son injection. La mort survient par oedème pulmonaire dû à l'hypertension artérielle induite principalement par la stimulation des récepteurs α-adrénergiques.

. L'administration préalable de substances α-adrénolytiques par voie orale permet de réduire la toxicité de la noradrénaline.

Les produits B 1020 - B 1021 - B 1037 - B 1059 sont administrés par voie orale 30 min avant l'injection intraveineuse de noradrénaline (0,4 mg/kg).

b) Résultats

Les résultats sont rapportés dans le tableau 3.

TABLEAU 3

| Produits | Dose en mg/kg P.O | % de protection contre la mort |
|---|---|---|
| B 1020 | 50 | 80 |
| B 1021 | 50 | 40 |
| B 1037 | 50 | 50 |
| B 1059 | 50 | 50 |
| Thymoxamine | 50 | 80 |

Ces résultats montrent qu'administrés à la dose de 50 mg/kg per os chez le rat les produits testés exercent une protection efficace contre la toxicité de la noradrénaline.

4. Spécificité urétrale de l'activité alpha bloquante déterminée in vivo chez le lapin :

a) - Principe de la mesure

. La noradérnaline (I.V.) provoque des augmentations dépendantes des doses de la pression artérielle et de la pression urétrale. Les substances adrénolytiques alpha antagonisent ces augmentations de pression.

. Pour les produits B 1020 - B 1037 et B 1059 administrés par voie veineuse, nous avons déterminé les doses inhibant de 50 p. cent ($DI_{50}$) l'effet de la noradrénaline au niveau artériel et urétral et évaluer le rapport R de ces $DI_{50}$ .

b) - Résultats obtenus

Les résultats sont consignés dans le Tableau 4.

Tableau 4

| | Doses ($mg.Kg^{-1}$) inhibant de 50 p.cent ($DI_{50}$) l'effet hypertenseur de la noradrénaline au niveau : | | R |
|---|---|---|---|
| | artériel $DI_{50}$ (a) | urétral $DI_{50}$ (u) | $\frac{DI50\ (a)}{DI50\ (u)}$ |
| B 1020 | > 3 | 1,7 | > 1,7 |
| B 1037 | > 3 | 0,5 | > 6 |
| B 1059 | > 3 | 0,8 | > 3,7 |
| THYMOXAMINE | 2,9 | 0,7 | 4,1 |

Ces résultats montrent l'efficacité plus importante de B 1020- B1037 - B 1059 au niveau urétral qu'au niveau vasculaire vis-à-vis de l'effet contracturant de la noradrénaline sur la musculature lisse de ces tissus. Ils laissent présumer d'une bonne efficacité (sans effet vasculaire important) dans le traitement des dysuries liés à une hypertonie urétrale par administration orale ou intra-veineuse.

**Revendications**

1. Produits chimiques de formule :

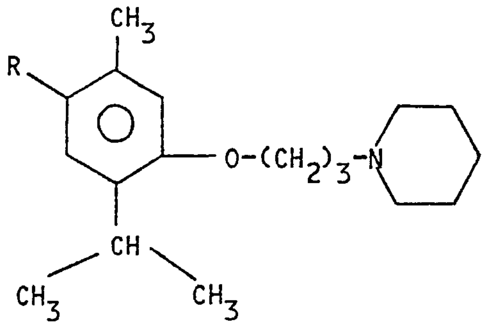

(I)

dans laquelle R est un radical choisi parmi H, acétyl ($COCH_3$), acétoxy ($OCOCH_3$) et hydroxy (OH), ainsi que les sels desdits produits, avec des acides, en vue de la préparation d'un composé pharmaceutiquement acceptable.

2. Procédé de préparation d'un produit de formule (I) dans lequel R est H, caractérisé en ce que l'on fait réagir le thymol avec la N-(chloro-3 propyl)-pipéridine, ladite réaction étant réalisée par transfert de phase liquide-liquide en présence d'un catalyseur tel que le chlorure de triéthylbenzylammonium.

3. Procédé de préparation d'un produit de formule (I) dans lequel R est acétyle, caractérisé en ce que l'on fait réagir un produit de formule (I) dans lequel R est H avec de l'anhydride acétique en présence d'acide perchlorique.

4. Procédé de préparation d'un produit de formule (I) dans lequel R est acétoxy, caractérisé en ce que l'on fait réagir un produit de formule (I) dans lequel R est acétyle avec l'acide m-chloroperbenzoïque en présence d'un acide.

5. Procédé de préparation d'un produit de formule (I) dans lequel R est hydroxy, caractérisé en ce que l'on saponifie un produit de formule (I) dans lequel R est acétoxy à l'aide de préférence d'une solution sodique.

6. Médicaments, caractérisés en ce qu'ils comportent au moins un produit selon la revendication 1.

Revendications pour les Etats contractants suivant: AT, GR, ES

1. Procédé de préparation de produits chimiques de formule

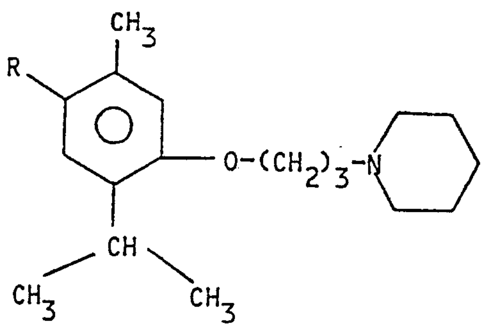

dans laquelle R est un radical choisi parmi H, acétyl ($COCH_3$), acétoxy ($OCOCH_3$) et hydroxy (OH), ainsi que les sels desdits produits, avec des acides, en vue de la préparatlon d'un composé pharmaceutiquement acceptable, caractérisé en ce que l'on prépare d'ahrd un produit de formule I dans lequel R est H en faisant réagir le thymol avec la N-(chloro-3 propyl)-pipéridine, ladite réaction étant realisée par transfert de phase liquide-liquide en présence d'un catalyseur tel que le chlorure de triéthyl-benzylammonium puis, éventuellement, on réalise les produits de formu1e (I) pour 1esquels R est acétyl, acétoxy et hydroxy.

2. Procédé de préparation d'un produit de formule (I) dans lequel R est acétyle, caractérisé en ce que l'on fait réagir un produit de formule (I) dans lequel R est H avec de l'anhydride acétique en présence d'acide perchlorique.

3. Procédé de préparation d'un produit de formule (I) dans lequel R est acétoxy, caractérisé en ce que l'on fait réagir un produit de formule (I) dans lequel R est acétyle avec l'acide m-chloroperbenzoïque en présence d'un acide.

4. Procédé de préparation d'un produit de formule (I) dans lequel R est hydroxy, caractérisé en ce que l'on saponifie un produit de formule (I) dans lequel R est acétoxy à l'aide de préférence d'une solution sodique.